# EUROPEAN PATENT SPECIFICATION

(11) **EP 2 970 830 B1**
(45) Date of publication and mention of the grant of the patent: **13.12.2017**
(21) Application number: 14719981.4
(22) Date of filing: 14.03.2014
(51) Int. Cl.: C11D 3/386, C07K 5/04, C12N 9/64, B65D 65/46, C12N 9/96, C11D 17/04

(54) **ENZYME AND INHIBITOR CONTAINED IN WATER-SOLUBLE FILMS**
PROTEASE UND HEMMER ENTHALTEND IN WASSERLÖSLICHEN FILMEN
FILMS SOLUBLES DANS L'EAU CONTENANT UNE PROTÉASE ET UN INHIBITEUR

(30) Priority: 14.03.2013 US 201361782721 P
(43) Date of publication of application: 20.01.2016
(73) Proprietor: Novozymes A/S, 2880 Bagsvaerd (DK); Novozymes North America, Inc., Franklinton, North Carolina 27525 (US); Monosol, LLC, Merrillville, Indiana 46410 (US)
(72) Inventor: SIMONSEN, Ole, DK-2860 Soeborg (DK); CASELLA, Victor, Raleigh, North Carolina 27616 (US); LEE, David, Crown Point, Indiana 46307 (US); CHILDERS, Jennifer, Lowell, Indiana 46356 (US)
(74) Representative: Bauditz, Peter
(86) International application number: PCT/US2014/027603
(87) International publication number: WO 2014/152674

(56) References cited:
- WO-A1-2011/094470
- WO-A1-2013/004636
- WO-A2-2004/031271
- US-A- 4 115 292
- US-A- 4 537 707
- OLSEN ET AL: "Enzyme-based antifouling coatings", BIOFOULING (CHUR), HARWOOD ACADEMIC PUBLISHERS, CHUR, CH, vol. 23, no. 5/6, 1 January 2007 (2007-01-01), pages 369-383, XP009137736, ISSN: 0892-7014
- NADEZHDA G BALABUSHEVITCH ET AL: "Regulating aspects of biosoluble and insoluble film release systems containing protein proteinase inhibitor", APPLIED BIOCHEMISTRY AND BIOTECHNOLOGY ; PART A: ENZYME ENGINEERING AND BIOTECHNOLOGY, HUMANA PRESS INC, NEW YORK, vol. 61, no. 1 - 2, 1 October 1996 (1996-10-01), pages 129-138, XP035177065, ISSN: 1559-0291, DOI: 10.1007/BF02785695
- GANZ P J ET AL: "Stabilized variant of Streptomyces subtilisin inhibitor and its use in stabilizing subtilisin BPN", PROTEIN ENGINEERING, DESIGN AND SELECTION, OXFORD JOURNAL, LONDON, GB, vol. 17, no. 4, 1 June 2004 (2004-06-01), pages 333-339, XP007903774, ISSN: 1741-0126, DOI: 10.1093/PROTEIN/GZH045

## Description

### BACKGROUND OF THE INVENTION

### Field of the Invention

The present invention relates to enzymatic water-soluble films comprising protease inhibitors, and their use in detergents.

### Background

The use of water-soluble film packages to deliver unit dosage amounts of detergents products for e.g. laundry and automatic dish wash is well known (see *e.g.,* WO 2009/098660 or WO 2010/141301). Both granular and liquid detergents have been on the market in this form for several years. It is also well known for decades to use enzymes in laundry detergents. More and more different types of enzymes are used in detergents, and the dosages of the enzymes is also increasing, amongst others due to the benefits coming from the enzymes and the environmental benefits of using biological actives instead of e.g. oil based chemicals like most surfactants.

A potential problem when using enzymes in detergents is the storage stability of the enzymes. Enzymes are large biological molecules that can undergo various forms of degradation. To overcome this problem numerous solutions have been suggested and patented, involving both designing more robust enzymes, and making detergent formulations less harsh to the enzymes. For unit dose systems like detergent pouches or tablets it is often useful to separate the enzymes from more harsh chemicals (e.g., bleach) in different compartments or layers. However, this is complicating the manufacturing processes and increases the cost. It has previously been suggested to incorporate the enzymes into the water-soluble film surrounding a detergent pouch (e.g., US 4,115,292).

Even when enzymes are protected from chemicals in the detergent composition, other enzymes in the film - in particular proteases - may influence stability of the enzymes during storage. Storage stability is not the only issue. During production of the film, the enzymes are active in the liquid used for preparing (casting) the film, and proteolytic activity exhibited by proteases will negatively influence the residual activity of all enzymes in the final film product. This is of particular importance when film offcuts from the cutting step is processed for reuse in the casting of new film.

The present invention provides a solution for both increasing the storage stability of enzymes in a water-soluble film, and for increasing the residual activity of enzymes in a water-soluble film.

### SUMMARY OF THE INVENTION

In a first aspect, the present invention provides a water-soluble film comprising a protease and a protease inhibitor.

Various other aspects and embodiments are apparent from the detailed description, examples and claims.

### DETAILED DESCRIPTION OF THE INVENTION

The inventors of the present invention have surprisingly found that the storage stability of enzymes incorporated in a water-soluble film, which contains at least one protease, can be improved by including a protease inhibitor in the film. Even though the water activity in the film is very low, and therefore the enzymatic activity is also low, the protease activity influences the storage stability of both proteases and other enzymes in the film. This is a surprising discovery, because proteases (and other hydrolytic enzymes) need water to exhibit proteolytic (hydrolytic) activity.

Inclusion of a protease inhibitor in the water-soluble film will further protect protease sensitive components, as proteins or peptide based materials in the detergent composition, against the protease activity exhibited at the interface between film and detergent composition.

In addition, the inventors have found that by including a protease inhibitor in a water-soluble film, which contains at least one protease, the loss of enzymatic activity during production of the film can be reduced. During production, the film is prepared from a liquid composition, wherein the protease exhibits proteolytic activity towards other proteases (autoproteolysis) and towards other enzymes and proteins. Therefore, the invention provides a higher residual activity of the enzymes in the water-soluble film after production.

### Enzymes

The enzyme(s) comprised in the enzyme and protease inhibitor containing water-soluble film of the invention include at least one protease, and optionally one or more (other) enzymes such as a protease, lipase, cutinase, amylase, carbohydrase, cellulase, pectinase, mannanase, arabinase, galactanase, xylanase, perhydrolase, oxidase, e.g., laccase, peroxidase and/or haloperoxidase.

Proteases: The proteases for use in the present invention may be serine proteases, such as subtilisins, and/or trypsin-like proteases; or metalloproteases. Preferably, the proteases are subtilisins.

A serine protease may for example be of the S1 family, such as trypsin, or the S8 family such as subtilisin. A metalloprotease may for example be a thermolysin from, e.g., family M4 or other metalloprotease, such as those from M5, M7 or M8 families.

A serine protease is an enzyme which catalyzes the hydrolysis of peptide bonds, and in which there is an essential serine residue at the active site (White, Handler and Smith, 1973 "Principles of Biochemistry," Fifth Edition, McGraw-Hill Book Company, NY, pp. 271-272). Subtilisins include, preferably consist of, the I-S1 and I-S2 sub-groups as defined by Siezen et al., Protein Engng. 4 (1991) 719-737; and Siezen et al., Protein Science 6 (1997) 501-523. Because of the highly conserved structure of the active site of serine proteases, the subtilisin according to the invention may be functionally equivalent to the proposed sub-group designated subtilase by Siezen *et al.* (supra).

The subtilisin may be of animal, vegetable or microbial origin, including chemically or genetically modified mutants (protein engineered variants), preferably an alkaline microbial subtilisin. Examples of subtilisins are those derived from *Bacillus, e.g.,* subtilisin Novo, subtilisin Carlsberg, subtilisin BPN', subtilisin 309, subtilisin 147 and subtilisin 168 (described in WO 89/06279) and Protease PD138 (WO 93/18140). Examples are described in WO 98/020115, WO 01/44452, WO 01/58275, WO 01/58276, WO 03/006602 and WO 04/099401. Examples of trypsin-like proteases are trypsin (e.g., of porcine or bovine origin) and the *Fusarium* protease described in WO 89/06270 and WO 94/25583. Other examples are the variants described in WO 92/19729, WO 88/08028, WO 98/20115, WO 98/20116, WO 98/34946, WO 2000/037599, WO 2011/036263, especially the variants with substitutions in one or more of the following positions: 27, 36, 57, 76, 87, 97, 101, 104, 120, 123, 167, 170, 194, 206, 218, 222, 224, 235, and 274.

Examples of commercially available protease, most of which are subtilisins, include Kannase™, Everlase™, Relase™, Esperase™, Alcalase™, Durazym™, Savinase™, Ovozyme™, Liquanase™, Coronase™, Polarzyme™, Pyrase™, Neutrase™, Pancreatic Trypsin NOVO (PTN), Bio-Feed™ Pro, Clear-Lens™ Pro, and Blaze (all available from Novozymes A/S, Bagsvaerd, Denmark). Other commercially available proteases include Ronozyme™ Pro, Maxatase™, Maxacal™, Maxapem™, Opticlean™, Properase™, Purafast™, Purafect™, Purafect Ox™, Purafact Prime™, Excellase™, FN2™, FN3™ and FN4™ (available from Genencor International Inc., DuPont, Gist-Brocades, BASF, or DSM). Other examples are Primase™ and Duralase™. Blap R, Blap S and Blap X available from Henkel are also examples.

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having color care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme™, Carezyme™, and Celluclean™ products (Novozymes A/S); Clazinase™ and Puradax HA™ (Genencor International Inc.), and KAC-500(B)™ (Kao Corporation).

Lipases and Cutinases: Suitable lipases and cutinases include those of bacterial or fungal origin. Chemically modified or protein engineered mutants are included. Examples include lipase from *Thermomyces, e.g.,* from *T. lanuginosus* (previously named *Humicola lanuginosa*) as described in EP 258 068 and EP 305 216, cutinase from *Humicola, e.g. H. insolens* as described in WO 96/13580, a *Pseudomonas* lipase, *e.g.,* from *P. alcaligenes* or *P*. *pseudoalcaligenes* (EP 218 272), *P. cepacia* (EP 331 376), *P. stutzeri* (GB 1,372,034), *P*. *fluorescens, Pseudomonas sp.* strain SD 705 (WO 95/06720 and WO 96/27002), *P*. *wisconsinensis* (WO 96/12012), a *Bacillus* lipase, *e.g.,* from *B. subtilis* (Dartois et al., 1993, Biochemica et Biophysica Acta, 1131: 253-360), *B. stearothermophilus* (JP 64/744992) or *B. pumilus* (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079, WO 97/07202, WO 00/060063, WO2007/087508 and WO 2009/109500.

Preferred commercially available lipase enzymes include Lipolase™, Lipolase Ultra™, Lipex™, Lipex Evity™, Calipso™, Lecitase™, Lipolex™, Lipoclean™, Lipoprime™ (Novozymes A/S). Other commercially available lipases include Lumafast (Genencor Int Inc); Lipomax (Gist-Brocades/Genencor Int Inc) and Bacillus sp. lipase from Solvay.

Amylases: Suitable amylases (α and/or β) include those of bacterial or fungal origin. Chemically modified or protein engineered mutants are included. Amylases include, for example, α-amylases obtained from *Bacillus, e.g.,* a special strain of *Bacillus licheniformis,* described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are Duramyl™, Termamyl™, Stainzyme™, Stainzyme Plus™, Resiliance™, Everest™, Natalase™, Fungamyl™, and BAN™ (Novozymes A/S); Rapidase™ and Purastar™ (from Genencor International Inc.).

Oxidases/peroxidases: Suitable oxidases and peroxidases (or oxidoreductases) include various sugar oxidases, laccases, peroxidases and haloperoxidases.

Suitable peroxidases include those comprised by the enzyme classification EC 1.11.1.7, as set out by the Nomenclature Committee of the International Union of Biochemistry and Molecular Biology (IUBMB), or any fragment derived therefrom, exhibiting peroxidase activity.

Suitable peroxidases include those of plant, bacterial or fungal origin. Chemically modified or protein engineered mutants are included. Examples of useful peroxidases include peroxidases from *Coprinopsis, e.g.,* from *C. cinerea* (EP 179,486), and variants thereof as those described in WO 93/24618, WO 95/10602, and WO 98/15257.

A peroxidase for use in the invention also include a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions.

In an embodiment, the haloperoxidase is a chloroperoxidase. Preferably, the haloperoxidase is a vanadium haloperoxidase, *i.e.,* a vanadate-containing haloperoxidase. In a preferred method of the present invention the vanadate-containing haloperoxidase is combined with a source of chloride ion.

Haloperoxidases have been isolated from many different fungi, in particular from the fungus group dematiaceous hyphomycetes, such as *Caldariomyces, e.g., C. fumago, Alternaria, Curvularia, e.g., C. verruculosa* and *C. inaequalis, Drechslera, Ulocladium* and *Botrytis.*

Haloperoxidases have also been isolated from bacteria such as *Pseudomonas, e.g., P. pyrrocinia* and *Streptomyces, e.g., S. aureofaciens.*

In an preferred embodiment, the haloperoxidase is derivable from *Curvularia* sp., in particular *Curvularia verruculosa* or *Curvularia inaequalis,* such as *C. inaequalis* CBS 102.42 as described in WO 95/27046; or *C. verruculosa* CBS 147.63 or *C. verruculosa* CBS 444.70 as described in WO 97/04102; or from *Drechslera hartlebii* as described in WO 01/79459, *Dendryphiella salina* as described in WO 01/79458, *Phaeotrichoconis crotalarie* as described in WO 01/79461, or *Geniculosporium* sp. as described in WO 01/79460.

An oxidase according to the invention include, in particular, any laccase enzyme comprised by the enzyme classification EC 1.10.3.2, or any fragment derived therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5).

Preferred laccase enzymes are enzymes of microbial origin. The enzymes may be derived from plants, bacteria or fungi (including filamentous fungi and yeasts).

Suitable examples from fungi include a laccase derivable from a strain of *Aspergillus, Neurospora, e.g., N. crassa, Podospora, Botrytis, Collybia, Fomes, Lentinus, Pleurotus, Trametes, e.g., T. villosa* and *T. versicolor, Rhizoctonia, e.g., R. solani, Coprinopsis, e.g., C. cinerea, C. comatus, C. friesii,* and *C. plicatilis, Psathyrella, e.g., P. condelleana, Panaeolus, e.g., P. papilionaceus, Myceliophthora, e.g., M. thermophila, Schytalidium, e.g., S. thermophilum, Polyporus, e.g., P. pinsitus, Phlebia, e.g., P. radiata* (WO 92/01046), or *Coriolus, e.g., C. hirsutus* (JP 2238885).

Suitable examples from bacteria include a laccase derivable from a strain of *Bacillus.*

A laccase derived from *Coprinopsis* or *Myceliophthora* is preferred; in particular a laccase derived from *Coprinopsis cinerea,* as disclosed in WO 97/08325; or from *Myceliophthora thermophila,* as disclosed in WO 95/33836.

Perhydrolase: Suitable perhydrolases are capable of catalyzing a perhydrolysis reaction that results in the production of a peracid from a carboxylic acid ester (acyl) substrate in the presence of a source of peroxygen (*e.g*., hydrogen peroxide). While many enzymes perform this reaction at low levels, perhydrolases exhibit a high perhydrolysis:hydrolysis ratio, often greater than 1. Suitable perhydrolases may be of plant, bacterial or fungal origin. Chemically modified or protein engineered mutants are included.

Examples of useful perhydrolases include naturally occurring *Mycobacterium* perhydrolase enzymes, or variants thereof. An exemplary enzyme is derived from *Mycobacterium smegmatis.* Such enzyme, its enzymatic properties, its structure, and variants thereof, are described in WO 2005/056782, WO 2008/063400, US 2008/145353, and US2007167344.

### Polyol

Polyols are often used as enzyme formulation agents, and may therefore eventually become a component of the water-soluble film, when a liquid enzyme formulation is used for preparing the water-soluble film. As described below under "Water-soluble film", polyols are also often used as plasticizers in water-soluble film.

A polyol (or polyhydric alcohol), when used as a component in the water-soluble film according to the invention, is an alcohol with two or more hydroxyl groups. The polyol typically includes less than 10 carbons, such as 9, 8, 7, 6, 5, 4, or 3 carbons. The molecular weight is typically less than 500 g/mol, such as 400 g/mol or 300 g/mol.

Examples of suitable polyols include, but are not limited to, glycerol, propylene glycol, ethylene glycol, diethylene glycol, hexylene glycol, sorbitol, mannitol, erythritol, dulcitol, inositol, xylitol and adonitol.

Generally, the water-soluble film of the invention includes less than 10% (w/w) polyol (polyhydric alcohol) per percent of active enzyme, i.e. the weight ratio of polyol to active enzyme is less than 10. Preferably, the weight ratio of polyol to active enzyme is less than 9, more preferably less than 8, more preferably less than 7, more preferably less than 6, more preferably less than 5, more preferably less than 4, most preferably less than 3, and in particular less than 2.

In an embodiment, the amount of polyol(s) in the water-soluble film is 10% to 50% (w/w), preferably 20% to 50% (w/w), more preferably 25% to 50% (w/w), even more preferably 25% to 45% (w/w), and most preferably 30% to 45% (w/w).

### Protease Inhibitor

A protease inhibitor is capable of reducing the proteolytic activity of a protease. The protease inhibitor according to the invention is a reversible inhibitor of protease activity, e.g., serine protease activity. Preferably, the protease inhibitor is a (reversible) subtilisin protease inhibitor. In particular, the protease inhibitor may be a peptide aldehyde, boric acid, or a boronic acid; or a derivative of any of these.

The inhibitor may have an inhibition constant to a serine protease Ki (M, mol/L) of from 1E-12 to 1E-03; more preferred from 1E-11 to 1E-04; even more preferred from 1E-10 to 1E-05; even more preferred from 1E-10 to 1E-06; and most preferred from 1E-09 to 1E-07.

The protease inhibitor according to the invention may be boronic acid or a derivative thereof; preferably, phenylboronic acid or a derivative thereof, as shown on pages 3-4 of WO96/41859, which is incorporated by reference. We have found that inclusion of boronic acids in the amounts needed in polyvinylalcohol based water soluble films does not create problems with solubility of the water-soluble film.

In an embodiment of the invention, the phenyl boronic acid derivative is of the following formula: 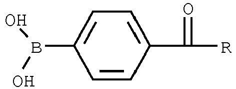 wherein R is selected from the group consisting of hydrogen, hydroxy, C₁-C₆ alkyl, substituted C₁-C₆ alkyl, C₁-C₆ alkenyl and substituted C₁-C₆ alkenyl. Preferably, R is hydrogen, CH₃, CH₃CH₂ or CH₃CH₂CH₂.

In a preferred embodiment, the protease inhibitor (phenyl boronic acid derivative) is 4-formyl-phenyl-boronic acid (4-FPBA).

In another particular embodiment, the protease inhibitor is selected from the group consisting of:
thiophene-2 boronic acid, thiophene-3 boronic acid, acetamidophenyl boronic acid, benzofuran-2 boronic acid, naphtalene-1 boronic acid, naphtalene-2 boronic acid, 2-FPBA, 3-FBPA, 4-FPBA, 1-thianthrene boronic acid, 4-dibenzofuran boronic acid, 5-methylthiophene-2 boronic, acid, thionaphtrene boronic acid, furan-2 boronic acid, furan-3 boronic acid, 4,4 biphenyl-diborinic acid, 6-hydroxy-2-naphtalene, 4-(methylthio) phenyl boronic acid, 4 (trimethylsilyl)phenyl boronic acid, 3-bromothiophene boronic acid, 4-methylthiophene boronic acid, 2-naphtyl boronic acid, 5-bromothiphene boronic acid, 5-chlorothiophene boronic acid, dimethylthiophene boronic acid, 2-bromophenyl boronic acid, 3-chlorophenyl boronic acid, 3-methoxy-2-thiophene, p-methyl-phenylethyl boronic acid, 2-thianthrene boronic acid, dibenzothiophene boronic acid, 4-carboxyphenyl boronic acid, 9-anthryl boronic acid, 3,5 dichlorophenyl boronic, acid, diphenyl boronic acidanhydride, o-chlorophenyl boronic acid, p-chlorophenyl boronic acid, m-bromophenyl boronic acid, p-bromophenyl boronic acid, p-flourophenyl boronic acid, p-tolyl boronic acid, o-tolyl boronic acid, octyl boronic acid, 1,3,5 trimethylphenyl boronic acid, 3-chloro-4-flourophenyl boronic acid, 3-aminophenyl boronic acid, 3,5-bis-(triflouromethyl) phenyl boronic acid, 2,4 dichlorophenyl boronic acid, 4-methoxyphenyl boronic acid.

Further boronic acid derivatives suitable as protease inhibitors according to the invention are described in US 4,963,655, US 5,159,060, WO 95/12655, WO 95/29223, WO 92/19707, WO 94/04653, WO 94/04654, US 5442100, US 5488157 and US 5472628.

The protease inhibitor according to the invention may also be a peptide aldehyde, as shown on pages 2-5 of WO2009/118375 (which is incorporated by reference), having the formula X-B¹-B⁰-H, wherein the groups have the following meaning:
a) H is hydrogen;
b) B⁰ is a single amino acid residue with L- or D-configuration and with the formula: NH-CHR-CO;
c) B¹ is a single amino acid residue; and
d) X consists of one or more amino acid residues (preferably one or two), optionally comprising an N-terminal protection group.

NH-CHR-CO (B⁰) is an L or D-amino acid residue, where R may be an aliphatic or aromatic side chain, *e.g.,* aralkyl, such as benzyl, where R may be optionally substituted. More particularly, the B⁰ residue may be bulky, neutral, polar, hydrophobic and/or aromatic. Examples are the D- or L-form of Tyr (*p*-tyrosine), *m*-tyrosine, 3,4-dihydroxyphenylalanine, Phe, Val, Met, norvaline (Nva), Leu, Ile or norleucine (Nle).

In the above formula, X-B¹-B⁰-H, the B¹ residue may particularly be small, aliphatic, hydrophobic and/or neutral. Examples are alanine (Ala), cysteine (Cys), glycine (Gly), proline (Pro), serine (Ser), threonine (Thr), valine (Val), norvaline (Nva) and norleucine (Nle), particularly alanine, glycine, or valine.

X may in particular be one or two amino acid residues with an optional N-terminal protection group (i.e. the compound is a tri- or tetrapeptide aldehyde with or without a protection group). Thus, X may be B², B³-B², Z-B², or Z-B³-B² where B³ and B² each represents one amino acid residue, and Z is an N-terminal protection group. The B² residue may in particular be small, aliphatic and/or neutral, e.g., Ala, Gly, Thr, Arg, Leu, Phe or Val. The B³ residue may in particular be bulky, hydrophobic, neutral and/or aromatic, *e.g.,* Phe, Tyr, Trp, Phenylglycine, Leu, Val, Nva, Nle or Ile.

The N-terminal protection group Z (if present) may be selected from formyl, acetyl, benzoyl, trifluoroacetyl, fluoromethoxy carbonyl, methoxysuccinyl, aromatic and aliphatic urethane protecting groups, benzyloxycarbonyl (Cbz), t-butyloxycarbonyl, adamantyloxycarbonyl, p-methoxybenzyl carbonyl (MOZ), benzyl (Bn), p-methoxybenzyl (PMB) or p-methoxyphenyl (PMP), methoxycarbonyl (Moc); methoxyacetyl (Mac); methyl carbamate or a methylamino carbonyl/methyl urea group. In the case of a tripeptide aldehyde with a protection group (i.e. X = Z-B²), Z is preferably a small aliphatic group, e.g., formyl, acetyl, fluoromethoxy carbonyl, t-butyloxycarbonyl, methoxycarbonyl (Moc); methoxyacetyl (Mac); methyl carbamate or a Methylamino carbonyl/methyl urea group. In the case of a tripeptide aldehyde with a protection group (i.e. X = Z-B³-B²), Z is preferably a bulky aromatic group such as benzoyl, benzyloxycarbonyl, p-methoxybenzyl carbonyl (MOZ), benzyl (Bn), p-methoxybenzyl (PMB) or p-methoxyphenyl (PMP).

Suitable peptide aldehydes are described in WO 94/04651, WO 95/25791, WO 98/13458, WO 98/13459, WO 98/13460, WO 98/13461, WO 98/13461, WO 98/13462, WO 2007/141736, 2007/145963, WO 2009/118375, WO 2010/055052 and WO 2011/036153. More particularly, the peptide aldehyde may be Cbz-RAY-H, Ac-GAY-H, Cbz-GAY-H, Cbz-GAL-H, Cbz-VAL-H, Cbz-GAF-H, Cbz-GAV-H, Cbz-GGY-H, Cbz-GGF-H, Cbz-RVY-H, Cbz-LVY-H, Ac-LGAY-H, Ac-FGAY-H, Ac-YGAY-H, Ac-FGAL-H, Ac-FGAF-H, Ac-FGVY-H, Ac-FGAM-H, Ac-WLVY-H, MeO-CO-VAL-H, MeNCO-VAL-H, MeO-CO-FGAL-H, MeO-CO-FGAF-H, MeSO₂-FGAL-H, MeSO₂-VAL-H, PhCH₂O(OH)(O)P-VAL-H, EtSO₂-FGAL-H, PhCH₂SO₂-VAL-H, PhCH₂O(OH)(O)P-LAL-H, PhCH₂O(OH)(O)P-FAL-H, or MeO(OH)(O)P-LGAL-H. Here, Cbz is benzyloxycarbonyl, Me is methyl, Et is ethyl, Ac is acetyl, H is hydrogen, and the other letters represent amino acid residues denoted by standard single letter notification (e.g., F = Phe, Y = Tyr, L = Leu).

Alternatively, the peptide aldehyde may have the formula as described in WO 2011/036153, which is incorporated by reference:

P-O-(Aᵢ-X')ₙ-Aₙ₊₁-Q

wherein Q is hydrogen, CH₃, CX₃, CHX₂, or CH₂X, wherein X is a halogen atom;
wherein one X' is the "double N-capping group" CO, CO-CO, CS, CS-CS or CS-CO, most preferred urido (CO), and the other X' es are nothing,
wherein n = 1-10, preferably 2-5, most preferably 2,
wherein each of Aᵢ and Aₙ₊₁ is an amino acid residue having the structure:
   - NH-CR-CO- for a residue to the right of X= -CO-, or
   - CO-CR-NH- for a residue to the left of X= -CO-
wherein R is H- or an optionally substituted alkyl or alkylaryl group which may optionally include a hetero atom and may optionally be linked to the N atom, and
wherein P is hydrogen or any C-terminal protection group.
Examples of such peptide aldehydes include α-MAPI, β-MAPI, F-urea-RVY-H, F-urea-GGY-H, F-urea-GAF-H, F-urea-GAY-H, F-urea-GAL-H, F-urea-GA-Nva-H, F-urea-GA-Nle-H, Y-urea-RVY-H, Y-urea-GAY-H, F-CS-RVF-H, F-CS-RVY-H, F-CS-GAY-H, Antipain, GE20372A, GE20372B, Chymostatin A, Chymostatin B, and Chymostatin C. Further examples of peptide aldehydes are disclosed in WO 2010/055052 and WO 2009/118375, WO 94/04651, WO 98/13459, WO 98/13461, WO 98/13462, WO 2007/145963, (P&G) hereby incorporated by reference.

Alternatively to a peptide aldehyde, the protease inhibitor may be a hydrosulfite adduct, as described in WO2013/004636 (which is incorporated by reference), having the formula X-B¹-NH-CHR-CHOH-SO₃M, wherein X, B¹ and R are defined as above, and M is H or an alkali metal, preferably Na or K.

The peptide aldehyde may be converted into a water-soluble hydrosulfite adduct by reaction with sodium bisulfite, as described in textbooks, *e.g.,* March, J. Advanced Organic Chemistry, fourth edition, Wiley-Interscience, US 1992, p 895.

An aqueous solution of the bisulfite adduct may be prepared by reacting the corresponding peptide aldehyde with an aqueous solution of sodium bisulfite (sodium hydrogen sulfite, NaHSO₃); potassium bisulfite (KHSO₃) by known methods, *e.g.,* as described in WO 98/47523; US 6,500,802; US 5,436,229; J. Am. Chem. Soc. (1978) 100, 1228; Org. Synth., Coll. vol. 7: 361.

The molar ratio of the above-mentioned peptide aldehydes (or hydrosulfite adducts) to the protease may be at least 1:1 or 1.5:1, and it may be less than 1000:1, more preferred less than 500:1, even more preferred from 100:1 to 2:1 or from 20:1 to 2:1, or most preferred, the molar ratio is from 10:1 to 2:1.

Formate salts (*e.g*., sodium formate) and formic acid have also shown good effects as inhibitor of protease activity. Formate can be used synergistically with the above-mentioned protease inhibitors, as shown in WO 2013/004635. Combinations of salts of formate with the above-mentioned protease inhibitors, as disclosed in WO 2013/004635, are incorporated by reference as a "protease inhibitor" for use in the invention. The formate salts may be present in the film in an amount of at least 0.1 % w/w or 0.5% w/w, *e.g.,* at least 1.0%, at least 1.2% or at least 1.5%. The amount of the salt is typically below 5% w/w, below 4% or below 3%.

In an embodiment of this invention the protease is a metalloprotease and the inhibitor is a metalloprotease inhibitor, *e.g.,* a protein hydrolysate based inhibitor (*e.g.*, as described in WO 2008/134343).

### Water-soluble film

Water-soluble films, optional ingredients for use therein, and methods of making the same are well known in the art. In one class of embodiments, the water-soluble film includes PVOH. PVOH is a synthetic resin generally prepared by the alcoholysis, usually termed hydrolysis or saponification, of polyvinyl acetate. Fully hydrolyzed PVOH, wherein virtually all the acetate groups have been converted to alcohol groups, is a strongly hydrogen-bonded, highly crystalline polymer which dissolves only in hot water - greater than about 140°F (60°C). If a sufficient number of acetate groups are allowed to remain after the hydrolysis of polyvinyl acetate, the PVOH polymer then being known as partially hydrolyzed, it is more weakly hydrogen-bonded and less crystalline and is soluble in cold water - less than about 50°F (10°C). An intermediate cold/hot water-soluble film can include, for example, intermediate partially-hydrolyzed PVOH (*e.g*., with degrees of hydrolysis of about 94% to about 98%), and is readily soluble only in warm water - *e.g.,* rapid dissolution at temperatures of about 40°C and greater. Both fully and partially hydrolyzed PVOH types are commonly referred to as PVOH homopolymers although the partially hydrolyzed type is technically a vinyl alcohol-vinyl acetate copolymer.

The degree of hydrolysis of the PVOH included in the water-soluble films of the present disclosure can be about 75% to about 99%. As the degree of hydrolysis is reduced, a film made from the resin will have reduced mechanical strength but faster solubility at temperatures below about 20°C. As the degree of hydrolysis increases, a film made from the resin will tend to be mechanically stronger and the thermoformability will tend to decrease. The degree of hydrolysis of the PVOH can be chosen such that the water-solubility of the resin is temperature dependent, and thus the solubility of a film made from the resin, compatibilizing agent, and additional ingredients is also influenced. In one class of embodiments the film is cold water-soluble. A cold water-soluble film, soluble in water at a temperature of less than 10°C, can include PVOH with a degree of hydrolysis in a range of about 75% to about 90%, or in a range of about 80% to about 90%, or in a range of about 85% to about 90%. In another class of embodiments the film is hot water-soluble. A hot water-soluble film, soluble in water at a temperature of at least about 60°C, can include PVOH with a degree of hydrolysis of at least about 98%.

Other film-forming resins for use in addition to or in an alternative to PVOH can include, but are not limited to, modified polyvinyl alcohols, polyacrylates, water-soluble acrylate copolymers, polyacrylates, polyacryamides, polyvinyl pyrrolidone, pullulan, water-soluble natural polymers including, but not limited to, guar gum, xanthan gum, carrageenan, and starch, water-soluble polymer derivatives including, but not limited to, ethoxylated starch and hydroxypropylated starch, poly(sodium acrylamido-2-methylpropane sulfonate), polymonomethylmaleate, copolymers thereof, and combinations of any of the foregoing. In one class of embodiments, the film-forming resin is a terpolymer consisting of vinyl alcohol, vinyl acetate, and sodium acrylamido-2-methylpropanesulfonate. Unexpectedly, water-soluble films based on a vinyl alcohol, vinyl acetate, and sodium acrylamido-2-methylpropanesulfonate terpolymer have demonstrated a high percent recovery of enzyme.

The water-soluble resin can be included in the water-soluble film in any suitable amount, for example an amount in a range of about 35 wt% to about 90 wt%. The preferred weight ratio of the amount of the water-soluble resin as compared to the combined amount of all enzymes, enzyme stabilizers, and secondary additives can be any suitable ratio, for example a ratio in a range of about 0.5 to about 5, or about 1 to 3, or about 1 to 2.

Water-soluble resins for use in the films described herein (including, but not limited to PVOH resins) can be characterized by any suitable viscosity for the desired film properties, optionally a viscosity in a range of about 5.0 to about 30.0 cP, or about 10.0 cP to about 25 cP. The viscosity of a PVOH resin is determined by measuring a freshly made solution using a Brookfield LV type viscometer with UL adapter as described in British Standard EN ISO 15023-2:2006 Annex E Brookfield Test method. It is international practice to state the viscosity of 4% aqueous polyvinyl alcohol solutions at 20°C. All PVOH viscosities specified herein in cP should be understood to refer to the viscosity of 4% aqueous polyvinyl alcohol solution at 20°C, unless specified otherwise.

It is well known in the art that the viscosity of a PVOH resin is correlated with the weight average molecular weight (*M̅w*) of the same PVOH resin, and often the viscosity is used as a proxy for *M̅w*. Thus, the weight average molecular weight of the water-soluble resin optionally can be in a range of about 35,000 to about 190,000, or about 80,000 to about 160,000. The molecular weight of the resin need only be sufficient to enable it to be molded by suitable techniques to form a thin plastic film.

The water-soluble films according to the present disclosure may include other optional additive ingredients including, but not limited to, plasticizers, surfactants, defoamers, film formers, antiblocking agents, internal release agents, anti-yellowing agents and other functional ingredients, for example in amounts suitable for their intended purpose.

Water is recognized as a very efficient plasticizer for PVOH and other polymers; however, the volatility of water makes its utility limited since polymer films need to have at least some resistance (robustness) to a variety of ambient conditions including low and high relative humidity. Glycerin is much less volatile than water and has been well established as an effective plasticizer for PVOH and other polymers. Glycerin or other such liquid plasticizers by themselves can cause surface "sweating" and greasiness if the level used in the film formulation is too high. This can lead to problems in a film such as unacceptable feel to the hand of the consumer and even blocking of the film on the roll or in stacks of sheets if the sweating is not mitigated in some manner, such as powdering of the surface. This could be characterized as over plasticization. However, if too little plasticizer is added to the film the film may lack sufficient ductility and flexibility for many end uses, for example to be converted into a final use format such as pouches.

Plasticizers for use in water-soluble films of the present disclosure include, but are not limited to, sorbitol, glycerol, diglycerol, propylene glycol, ethylene glycol, diethyleneglycol, triethylene glycol, tetraethyleneglycol, polyethylene glycols up to MW 400, 2 methyl 1, 3 propane diol, lactic acid, monoacetin, triacetin, triethyl citrate, 1,3-butanediol, trimethylolpropane (TMP), polyether triol, and combinations thereof. Polyols, as described above, are generally useful as plasticizers. As less plasticizer is used, the film can become more brittle, whereas as more plasticizer is used the film can lose tensile strength. Plasticizers can be included in the water-soluble films in an amount in a range of about 25 phr to about 50 phr, or from about 30 phr to about 45 phr, or from about 32 phr to about 42 phr, for example.

Surfactants for use in water-soluble films are well known in the art. Optionally, surfactants are included to aid in the dispersion of the resin solution upon casting. Suitable surfactants for water-soluble films of the present disclosure include, but are not limited to, dialkyl sulfosuccinates, lactylated fatty acid esters of glycerol and propylene glycol, lactylic esters of fatty acids, sodium alkyl sulfates, polysorbate 20, polysorbate 60, polysorbate 65, polysorbate 80, alkyl polyethylene glycol ethers, lecithin, acetylated fatty acid esters of glycerol and propylene glycol, sodium lauryl sulfate, acetylated esters of fatty acids, myristyl dimethylamine oxide, trimethyl tallow alkyl ammonium chloride, quaternary ammonium compounds, salts thereof and combinations of any of the forgoing. Too little surfactant can sometimes result in a film having holes, whereas too much surfactant can result in the film having a greasy or oily feel from excess surfactant present on the surface of the film. Thus, surfactants can be included in the water-soluble films in an amount of less than about 2 phr, for example less than about 1 phr, or less than about 0.5 phr, for example.

One type of secondary component contemplated for use is a defoamer. Defoamers can aid in coalescing of foam bubbles. Suitable defoamers for use in water-soluble films according to the present disclosure include, but are not limited to, hydrophobic silicas, for example silicon dioxide or fumed silica in fine particle sizes, including Foam Blast® defoamers available from Emerald Performance Materials, including Foam Blast® 327, Foam Blast® UVD, Foam Blast® 163, Foam Blast® 269, Foam Blast® 338, Foam Blast® 290, Foam Blast® 332, Foam Blast® 349, Foam Blast® 550 and Foam Blast® 339, which are proprietary, non-mineral oil defoamers. In embodiments, defoamers can be used in an amount of 0.5 phr, or less, for example, 0.05 phr, 0.04 phr, 0.03 phr, 0.02 phr, or 0.01 phr. Preferably, significant amounts of silicon dioxide will be avoided, in order to avoid stress whitening.

Processes for making water-soluble articles, including films, include casting, blow-molding, extrusion and blown extrusion, as known in the art. One contemplated class of embodiments is characterized by the water-soluble film described herein being formed by casting, for example, by admixing the ingredients described herein with water to create an aqueous mixture, for example a solution with optionally dispersed solids, applying the mixture to a surface, and drying off water to create a film. Similarly, other compositions can be formed by drying the mixture while it is confined in a desired shape.

In one contemplated class of embodiments, the water-soluble film is formed by casting a water-soluble mixture wherein the water-soluble mixture is prepared according to the steps of:
(a) providing a mixture of water-soluble resin, water, and any optional additives excluding plasticizers;
(b) boiling the mixture for 30 minutes;
(c) degassing the mixture in an oven at a temperature of at least 40°C; optionally in a range of 40°C to 70°C, e.g., about 65°C;
(d) adding one or more enzymes, plasticizer, and additional water to the mixture at a temperature of 65°C or less; and
(e) stirring the mixture without vortex until the mixture appears substantially uniform in color and consistency; optionally for a time period in a range of 30 minutes to 90 minutes, optionally at least 1 hour; or adding some or all of the enzymes as close to casting the film as possible, e.g., by adding the enzyme solution(s) to the film solution using in-line mixer/static mixer just before casting the film, thus reducing the storage period of the enzyme in the solution as much as possible; and
(f) casting the mixture promptly after the time period of stirring (e.g., within 4 hours, or 2 hours, or 1 hour, or even within minutes).

If the enzyme is added to the mixture too early, e.g., with the secondary additives or resin, the activity of the enzyme may decrease. Without intending to be bound by any particular theory, it is believed that boiling of the mixture with the enzyme leads to the enzyme denaturing and storing in solution for extended periods of time also leads to a reduction in enzyme activity.

In one class of embodiments, high enzyme activity is maintained in the water-soluble films according to the present disclosure by drying the films quickly under moderate to mild conditions. As used herein, drying quickly refers to a drying time of less than 24 hours, optionally less than 12 hours, optionally less than 8 hours, optionally less than 2 hours, optionally less than 1 hour, optionally less than 45 minutes, optionally less than 30 minutes, optionally less than 20 minutes, optionally less than 10 minutes, for example in a range of about 6 minutes to about 10 minutes, or 8 minutes. As used herein, moderate to mild conditions refer to drying temperatures of less than 170°F (77°C), optionally in a range of about 150°F to about 170°F (about 66°C to about 77°C), e.g., 165°F (74°C). As the drying temperature increases, the enzymes tend to denature faster, whereas as the drying temperature decreases, the drying time increases, thus exposing the enzymes to solution for an extended period of time.

The film is useful for creating a packet to contain a composition, for example laundry or dishwashing compositions, thereby forming a pouch. The film described herein can also be used to make a packet with two or more compartments made of the same film or in combination with films of other polymeric materials. Additional films can, for example, be obtained by casting, blow-molding, extrusion or blown extrusion of the same or a different polymeric material, as known in the art. In one type of embodiment, the polymers, copolymers or derivatives thereof suitable for use as the additional film are selected from polyvinyl alcohols, polyvinyl pyrrolidone, polyalkylene oxides, polyacrylic acid, cellulose, cellulose ethers, cellulose esters, cellulose amides, polyvinyl acetates, polycarboxylic acids and salts, polyaminoacids or peptides, polyamides, polyacrylamide, copolymers of maleic/acrylic acids, polysaccharides including starch and gelatin, natural gums such as xanthan, and carrageenans. For example, polymers can be selected from polyacrylates and water-soluble acrylate copolymers, methylcellulose, carboxymethylcellulose sodium, dextrin, ethylcellulose, hydroxyethyl cellulose, hydroxypropyl methylcellulose, maltodextrin, polymethacrylates, and combinations thereof, or selected from polyvinyl alcohols, polyvinyl alcohol copolymers and hydroxypropyl methyl cellulose (HPMC), and combinations thereof.

The pouches and/or packets of the present disclosure comprise at least one sealed compartment. Thus the pouches may comprise a single compartment or multiple compartments. The pouches may have regions with and without enzymes. In embodiments including multiple compartments, each compartment may contain identical and/or different compositions. In turn, the compositions may take any suitable form including, but not limited to liquid, solid and combinations thereof (e.g., a solid suspended in a liquid). In some embodiments, the pouches comprises a first, second and third compartment, each of which respectively contains a different first, second and third composition. In some embodiments, the compositions may be visually distinct as described in EP 2258820.

The compartments of multi-compartment pouches and/or packets may be of the same or different size(s) and/or volume(s). The compartments of the present multi-compartment pouches can be separate or conjoined in any suitable manner. In some embodiments, the second and/or third and/or subsequent compartments are superimposed on the first compartment. In one embodiment, the third compartment may be superimposed on the second compartment, which is in turn superimposed on the first compartment in a sandwich configuration. Alternatively the second and third compartments may be superimposed on the first compartment. However it is also equally envisaged that the first, second and optionally third and subsequent compartments may be attached to one another in a side by side relationship. The compartments may be packed in a string, each compartment being individually separable by a perforation line. Hence each compartment may be individually torn-off from the remainder of the string by the end-user,

In some embodiments, multi-compartment pouches and/or packets include three compartments consisting of a large first compartment and two smaller compartments. The second and third smaller compartments are superimposed on the first larger compartment. The size and geometry of the compartments are chosen such that this arrangement is achievable. The geometry of the compartments may be the same or different. In some embodiments the second and optionally third compartment each has a different geometry and shape as compared to the first compartment. In these embodiments, the second and optionally third compartments are arranged in a design on the first compartment. The design may be decorative, educative, or illustrative, for example to illustrate a concept or instruction, and/or used to indicate origin of the product. In some embodiments, the first compartment is the largest compartment having two large faces sealed around the perimeter, and the second compartment is smaller covering less than about 75%, or less than about 50% of the surface area of one face of the first compartment. In embodiments in which there is a third compartment, the aforementioned structure may be the same but the second and third compartments cover less than about 60%, or less than about 50%, or less than about 45% of the surface area of one face of the first compartment.

The pouches and/or packets of the present disclosure may comprise one or more different films. For example, in single compartment embodiments, the packet may be made from one wall that is folded onto itself and sealed at the edges, or alternatively, two walls that are sealed together at the edges. In multiple compartment embodiments, the packet may be made from one or more films such that any given packet compartment may comprise walls made from a single film or multiple films having differing compositions. In one embodiment, a multi-compartment pouch comprises at least three walls: an outer upper wall; an outer lower wall; and a partitioning wall. The outer upper wall and the outer lower wall are generally opposing and form the exterior of the pouch. The partitioning wall is interior to the pouch and is secured to the generally opposing outer walls along a seal line. The partitioning wall separates the interior of the multi-compartment pouch into at least a first compartment and a second compartment. In one class of embodiments, the partitioning wall may be the only enzyme containing film thereby minimizing the exposure of the consumer to the enzymes.

Pouches and packets may be made using any suitable equipment and method. For example, single compartment pouches may be made using vertical form filling, horizontal form filling, or rotary drum filling techniques commonly known in the art. Such processes may be either continuous or intermittent. The film may be dampened, and/or heated to increase the malleability thereof. The method may also involve the use of a vacuum to draw the film into a suitable mold. The vacuum drawing the film into the mold can be applied for about 0.2 to about 5 seconds, or about 0.3 to about 3, or about 0.5 to about 1.5 seconds, once the film is on the horizontal portion of the surface. This vacuum can be such that it provides an under-pressure in a range of 10 mbar to 1000 mbar, or in a range of 100 mbar to 600 mbar, for example.

The molds, in which packet s may be made, can have any shape, length, width and depth, depending on the required dimensions of the pouches. The molds may also vary in size and shape from one to another, if desirable. For example, the volume of the final pouches may be about 5 ml to about 300 ml, or about 10 to 150 ml, or about 20 to about 100 ml, and that the mold sizes are adjusted accordingly.

In one embodiment, the packet includes a first and a second sealed compartment. The second compartment is in a generally superposed relationship with the first sealed compartment such that the second sealed compartment and the first sealed compartment share a partitioning wall interior to the pouch.

In one embodiment, the packet including a first and a second compartment further includes a third sealed compartment. The third sealed compartment is in a generally superposed relationship with the first sealed compartment such that the third sealed compartment and the first sealed compartment share a partitioning wall interior to the pouch.

In various embodiments, the first composition and the second composition are selected from one of the following combinations: liquid, liquid; liquid, powder; powder, powder; and powder, liquid.

In various embodiments, the first, second and third compositions are selected from one of the following combinations: solid, liquid, liquid and liquid, liquid, liquid.

In one embodiment, the single compartment or plurality of sealed compartments contains a composition. The plurality of compartments may each contain the same or a different composition. The composition is selected from a liquid, solid or combination thereof.

Heat can be applied to the film in the process commonly known as thermoforming. The heat may be applied using any suitable means. For example, the film may be heated directly by passing it under a heating element or through hot air, prior to feeding it onto a surface or once on a surface. Alternatively, it may be heated indirectly, for example by heating the surface or applying a hot item onto the film. The film can be heated using an infrared light. The film may be heated to a temperature of at least 50°C, for example about 50 to about 150°C, about 50 to about 120°C, about 60 to about 130°C, about 70 to about 120°C, or about 60 to about 90°C.

Alternatively, the film can be wetted by any suitable means, for example directly by spraying a wetting agent (including water, a solution of the film composition, a plasticizer for the film composition, or any combination of the foregoing) onto the film, prior to feeding it onto the surface or once on the surface, or indirectly by wetting the surface or by applying a wet item onto the film.

Once a film has been heated and/or wetted, it may be drawn into an appropriate mold, preferably using a vacuum. The film can be thermoformed with a draw ratio of at least about 1.5, for example, and optionally up to a draw ratio of 2, for example. The filling of the molded film can be accomplished by utilizing any suitable means. In some embodiments, the most preferred method will depend on the product form and required speed of filling. In some embodiments, the molded film is filled by in-line filling techniques. The filled, open packets are then closed forming the pouches, using a second film, by any suitable method. This may be accomplished while in horizontal position and in continuous, constant motion. The closing may be accomplished by continuously feeding a second film, preferably water-soluble film, over and onto the open packets and then preferably sealing the first and second film together, typically in the area between the molds and thus between the packets.

Any suitable method of sealing the packet and/or the individual compartments thereof may be utilized. Non-limiting examples of such means include heat sealing, solvent welding, solvent or wet sealing, and combinations thereof. The water-soluble packet and/or the individual compartments thereof can be heat sealed at a temperature of at least 200°F (93°C), for example in a range of about 220°F (about 105°C) to about 290°F (about 145°C), or about 230°F (about 110°C) to about 280°F (about 140°C). Typically, only the area which is to form the seal is treated with heat or solvent. The heat or solvent can be applied by any method, typically on the closing material, and typically only on the areas which are to form the seal. If solvent or wet sealing or welding is used, it may be preferred that heat is also applied. Preferred wet or solvent sealing/welding methods include selectively applying solvent onto the area between the molds, or on the closing material, by for example, spraying or printing this onto these areas, and then applying pressure onto these areas, to form the seal. Sealing rolls and belts as described above (optionally also providing heat) can be used, for example.

The formed pouches may then be cut by a cutting device. Cutting can be accomplished using any known method. It may be preferred that the cutting is also done in continuous manner, and preferably with constant speed and preferably while in horizontal position. The cutting device can, for example, be a sharp item, or a hot item, or a laser, whereby in the latter cases, the hot item or laser 'burns' through the film/ sealing area.

The different compartments of a multi-compartment pouches may be made together in a side-by-side style wherein the resulting, cojoined pouches may or may not be separated by cutting. Alternatively, the compartments can be made separately.

In some embodiments, pouches may be made according to a process including the steps of:
a) forming a first compartment (as described above);
b) forming a recess within some or all of the closed compartment formed in step (a), to generate a second molded compartment superposed above the first compartment;
c) filling and closing the second compartments by means of a third film;
d) sealing the first, second and third films; and
e) cutting the films to produce a multi-compartment pouch.

The recess formed in step (b) may be achieved by applying a vacuum to the compartment prepared in step (a).

In some embodiments, second, and/or third compartment(s) can be made in a separate step and then combined with the first compartment as described in EP 2088187 or WO 2009/152031.

In other embodiments, pouches may be made according to a process including the steps of:
a) forming a first compartment, optionally using heat and/or vacuum, using a first film on a first forming machine;
b) filling the first compartment with a first composition;
c) on a second forming machine, deforming a second film, optionally using heat and vacuum, to make a second and optionally third molded compartment;
d) filling the second and optionally third compartments;
e) sealing the second and optionally third compartment using a third film;
f) placing the sealed second and optionally third compartments onto the first compartment;
g) sealing the first, second and optionally third compartments; and
h) cutting the films to produce a multi-compartment pouch.

The first and second forming machines may be selected based on their suitability to perform the above process. In some embodiments, the first forming machine is preferably a horizontal forming machine, and the second forming machine is preferably a rotary drum forming machine, preferably located above the first forming machine.

It should be understood that by the use of appropriate feed stations, it may be possible to manufacture multi-compartment pouches incorporating a number of different or distinctive compositions and/or different or distinctive liquid, gel or paste compositions.

### Detergent

The detergent, or detergent composition, which forms part of the present invention, may be a laundry detergent or a dish wash detergent composition, which is encapsulated in a compartment formed by, a protease and protease inhibitor containing water-soluble film, as described above; or which simply comprises a protease and protease inhibitor containing water-soluble film, as described above.

Preferably, the detergent composition is a liquid detergent composition, such as a liquid laundry or dish wash detergent composition, having a physical form, which is not solid. It may be a pourable liquid, a pourable gel or a non-pourable gel. It may be either isotropic or structured, preferably isotropic. It may be a formulation useful for washing in automatic washing machines or for hand washing.

Liquids, including without limitation, alkanols, amines, diols, ethers and polyols may be included in a liquid detergent. A liquid detergent may contain from 0-30% organic solvent. A liquid detergent may even be non-aqueous, or substantially non-aqueous, wherein the water content is below 15%, preferably below 10%, more preferably below 6%, more preferably below 4%, more preferably below 2%, and most preferably below 1%.

The detergent composition comprises one or more additional cleaning composition components. The choice of additional components is within the skill of the artisan and includes conventional ingredients, including the exemplary non-limiting components set forth below.

The choice of components may include, for textile care, the consideration of the type of textile to be cleaned, the type and/or degree of soiling, the temperature at which cleaning is to take place, and the formulation of the detergent product. Although components mentioned below are categorized by general header according to a particular functionality, this is not to be construed as a limitation, as a component may comprise additional functionalities as will be appreciated by the skilled artisan.

### Surfactants

The detergent composition may comprise one or more surfactants, which may be anionic and/or cationic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 0.1 % to 60% by weight, such as about 1% to about 40%, or about 3% to about 20%, or about 3% to about 10%. The surfactant(s) is chosen based on the desired cleaning application, and includes any conventional surfactant(s) known in the art. Any surfactant known in the art for use in detergents may be utilized.

When included therein the detergent will usually contain from about 1% to about 40% by weight, such as from about 5% to about 30%, including from about 5% to about 15%, or from about 20% to about 25% of an anionic surfactant. Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or soap, and combinations thereof.

When included therein the detergent will usually contain from about 0.1 % to about 10% by weight of a cationic surfactant. Non-limiting examples of cationic surfactants include alklydimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, and combinations thereof.

When included therein the detergent will usually contain from about 0.2% to about 40% by weight of a non-ionic surfactant, for example from about 0.5% to about 30%, in particular from about 1% to about 20%, from about 3% to about 10%, such as from about 3% to about 5%, or from about 8% to about 12%. Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxy alkyl fatty acid amides, or *N*-acyl N-alkyl derivatives of glucosamine (glucamides, GA, or fatty acid glucamide, FAGA), as well as products available under the trade names SPAN and TWEEN, and combinations thereof.

When included therein the detergent will usually contain from about 0.1 % to about 20% by weight of a semipolar surfactant. Non-limiting examples of semipolar surfactants include amine oxides (AO) such as alkyldimethylamineoxide, *N*-(coco alkyl)-*N,N*-dimethylamine oxide and *N-*(tallow-alkyl)-*N,N*-bis(2-hydroxyethyl)amine oxide, fatty acid alkanolamides and ethoxylated fatty acid alkanolamides, and combinations thereof.

When included therein the detergent will usually contain from about 0.1 % to about 10% by weight of a zwitterionic surfactant. Non-limiting examples of zwitterionic surfactants include betaine, alkyldimethylbetaine, sulfobetaine, and combinations thereof.

### Hydrotropes

A hydrotrope is a compound that solubilises hydrophobic compounds in aqueous solutions (or oppositely, polar substances in a non-polar environment). Typically, hydrotropes have both hydrophilic and a hydrophobic character (so-called amphiphilic properties as known from surfactants); however the molecular structure of hydrotropes generally do not favor spontaneous self-aggregation, see *e.g.,* review by Hodgdon and Kaler (2007), Current Opinion in Colloid & Interface Science 12: 121-128. Hydrotropes do not display a critical concentration above which self-aggregation occurs as found for surfactants and lipids forming miceller, lamellar or other well defined meso-phases. Instead, many hydrotropes show a continuous-type aggregation process where the sizes of aggregates grow as concentration increases. However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in detergent compositions allow for example more concentrated formulations of surfactants (as in the process of compacting liquid detergents by removing water) without inducing undesired phenomena such as phase separation or high viscosity.

The detergent may contain 0-5% by weight, such as about 0.5 to about 5%, or about 3% to about 5%, of a hydrotrope. Any hydrotrope known in the art for use in detergents may be utilized. Non-limiting examples of hydrotropes include sodium benzene sulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

### Builders and Co-Builders

The detergent composition may contain about 0-65% by weight, such as about 5% to about 50% of a detergent builder or co-builder, or a mixture thereof. In a dish wash detergent, the level of builder is typically 40-65%, particularly 50-65%. The builder and/or co-builder may particularly be a chelating agent that forms water-soluble complexes with Ca and Mg. Any builder and/or co-builder known in the art for use in laundry detergents may be utilized. Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (*e.g*., SKS-6 from Hoechst), ethanolamines such as 2-aminoethan-1-ol (MEA), diethanolamine (DEA, also known as iminodiethanol), triethanolamine (TEA, also known as 2,2',2"-nitrilotriethanol), and carboxymethyl inulin (CMI), and combinations thereof.

The detergent composition may also contain 0-50% by weight, such as about 5% to about 30%, of a detergent co-builder, or a mixture thereof. The detergent composition may include include a co-builder alone, or in combination with a builder, for example a zeolite builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-*N,N'-*disuccinic acid (EDDS), methylglycinediacetic acid (MGDA), glutamic acid-N,N-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), *N*-(2-hydroxyethyl)iminodiacetic acid (EDG), aspartic acid-*N*-monoacetic acid (ASMA), aspartic acid-N,N-diacetic acid (ASDA), aspartic acid-*N*-monopropionic acid (ASMP), iminodisuccinic acid (IDA), *N*-(2-sulfomethyl)-aspartic acid (SMAS), *N*-(2-sulfoethyl)-aspartic acid (SEAS), *N*-(2-sulfomethyl)-glutamic acid (SMGL), *N*-(2-sulfoethyl)-glutamic acid (SEGL), *N-*methyliminodiacetic acid (MIDA), α-alanine-*N*, *N*-diacetic acid (α-ALDA), serine-*N*, *N*-diacetic acid (SEDA), isoserine-*N*, *N*-diacetic acid (ISDA), phenylalanine-*N*, *N*-diacetic acid (PHDA), anthranilic acid-*N*, *N*-diacetic acid (ANDA), sulfanilic acid-*N*, *N*-diacetic acid (SLDA), taurine-*N*, *N*-diacetic acid (TUDA) and sulfomethyl-*N*, *N*-diacetic acid (SMDA), N-(2-hydroxyethyl)-ethylidenediamine-*N*, *N', N'*-triacetate (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, e.g., WO 2009/102854, US 5,977,053.

### Bleaching Systems

The detergent may contain 0-50% of a bleaching system. Any bleaching system known in the art for use in laundry detergents may be utilized. Suitable bleaching system components include bleaching catalysts, photobleaches, bleach activators, sources of hydrogen peroxide such as sodium percarbonate and sodium perborates, preformed peracids and mixtures thereof. Suitable preformed peracids include, but are not limited to, peroxycarboxylic acids and salts, percarbonic acids and salts, perimidic acids and salts, peroxymonosulfuric acids and salts, for example, Oxone (R), and mixtures thereof. Non-limiting examples of bleaching systems include peroxide-based bleaching systems, which may comprise, for example, an inorganic salt, including alkali metal salts such as sodium salts of perborate (usually mono- or tetra-hydrate), percarbonate, persulfate, perphosphate, persilicate salts, in combination with a peracid-forming bleach activator. The term bleach activator is meant herein as a compound which reacts with peroxygen bleach like hydrogen peroxide to form a peracid. The peracid thus formed constitutes the activated bleach. Suitable bleach activators to be used herein include those belonging to the class of esters amides, imides or anhydrides. Suitable examples are tetracetylethylene diamine (TAED), sodium 4-[(3,5,5-trimethylhexanoyl)oxy]benzene sulfonate (ISONOBS), diperoxy dodecanoic acid, 4-(dodecanoyloxy)benzenesulfonate (LOBS), 4-(decanoyloxy)benzenesulfonate, 4-(decanoyloxy)benzoate (DOBS), 4-(nonanoyloxy)-benzenesulfonate (NOBS), and/or those disclosed in WO 98/17767. A particular family of bleach activators of interest was disclosed in EP 624154 and particulary preferred in that family is acetyl triethyl citrate (ATC). ATC or a short chain triglyceride like triacetin has the advantage that it is environmental friendly as it eventually degrades into citric acid and alcohol. Furthermore acetyl triethyl citrate and triacetin has a good hydrolytical stability in the product upon storage and it is an efficient bleach activator. Finally ATC provides a good building capacity to the laundry additive. Alternatively, the bleaching system may comprise peroxyacids of, for example, the amide, imide, or sulfone type. The bleaching system may also comprise peracids such as 6-(phthalimido)peroxyhexanoic acid (PAP). The bleaching system may also include a bleach catalyst. In some embodiments the bleach component may be an organic catalyst selected from the group consisting of organic catalysts having the following formulae: 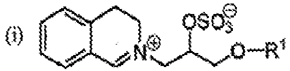 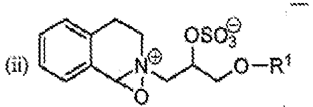 and mixtures thereof; wherein each R¹ is independently a branched alkyl group containing from 9 to 24 carbons or linear alkyl group containing from 11 to 24 carbons, preferably each R¹ is independently a branched alkyl group containing from 9 to 18 carbons or linear alkyl group containing from 11 to 18 carbons, more preferably each R¹ is independently selected from the group consisting of 2-propylheptyl, 2-butyloctyl, 2-pentylnonyl, 2-hexyldecyl, n-dodecyl, n-tetradecyl, n-hexadecyl, n-octadecyl, iso-nonyl, iso-decyl, iso-tridecyl and iso-pentadecyl. Other exemplary bleaching systems are described, *e.g.,* in WO 2007/087258, WO 2007/087244, WO 2007/087259 and WO 2007/087242. Suitable photobleaches may for example be sulfonated zinc phthalocyanine.

### Polymers

The detergent may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-N-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575. Salts of the above-mentioned polymers are also contemplated.

### Fabric hueing agents

The detergent compositions of the present invention may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO 2005/03274, WO 2005/03275, WO 2005/03276 and EP 1876226 (hereby incorporated by reference). The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, e.g., WO 2007/087257 and WO 2007/087243.

### (Additional) Enzymes

The detergent composition may comprise one or more (other) enzymes, in addition to the enzymes comprised in the water-soluble film. Examples of such enzymes are the same as those, which can be included in the enzyme containing water-soluble film, as shown above; for example protease, lipase, cutinase, amylase, carbohydrase, cellulase, pectinase, mannanase, arabinase, galactanase, xylanase, perhydrolase, oxidase, *e.g.,* laccase, peroxidase and/or haloperoxidase.

The detergent enzyme(s) may be included in a detergent composition by adding separate additives containing one or more enzymes, or by adding a combined additive comprising all of these enzymes. A detergent additive of the invention, *i.e.,* a separate additive or a combined additive, can be formulated, for example, as a granulate, liquid, slurry, etc. Preferred detergent additive formulations are granulates, in particular non-dusting granulates, liquids, in particular stabilized liquids, or slurries.

Non-dusting granulates may be produced, *e.g.,* as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protease inhibitors may, in addition to the water-soluble film, also be added to the detergent composition. Protected enzymes may be prepared according to the method disclosed in EP 238216.

### Adjunct materials

Any detergent components known in the art for use in laundry detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in laundry detergents may be utilized. The choice of such ingredients is well within the skill of the artisan.

Dispersants - The detergent compositions of the present invention can also contain dispersants. In particular powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

Dye Transfer inhibiting Agents - The detergent compositions of the present invention may also include one or more dye transfer inhibiting agents. Suitable polymeric dye transfer inhibiting agents include, but are not limited to, polyvinylpyrrolidone polymers, polyamine *N-*oxide polymers, copolymers of *N*-vinylpyrrolidone and *N*-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

Fluorescent whitening agent - The detergent compositions of the present invention will preferably also contain additional components that may tint articles being cleaned, such as fluorescent whitening agent or optical brighteners. Where present the brightener is preferably at a level of about 0.01% to about 0.5%. Any fluorescent whitening agent suitable for use in a laundry detergent composition may be used in the composition of the present invention. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino) stilbene-2.2'-disulfonate, 4,4'-bis-(2-anilino-4-(*N*-methyl-*N*-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2*H*-naphtho[1,2-*d*][1,2,3]triazol-2-yl)-2-[(*E*)-2-phenylvinyl]benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins.

Suitable fluorescent brightener levels include lower levels of from about 0.01, from 0.05, from about 0.1 or even from about 0.2 wt % to upper levels of 0.5 or even 0.75 wt%.

Soil release polymers - The detergent compositions of the present invention may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523 (hereby incorporated by reference). Furthermore random graft co-polymers are suitable soil release polymers. Suitable graft co-polymers are described in more detail in WO 2007/138054, WO 2006/108856 and WO 2006/113314 (hereby incorporated by reference). Other soil release polymers are substituted polysaccharide structures especially substituted cellulosic structures such as modified cellulose deriviatives such as those described in EP 1867808 or WO 2003/040279 (both are hereby incorporated by reference). Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

Anti-redeposition agents - The detergent compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

Rheology Modifiers are structurants or thickeners, as distinct from viscosity reducing agents. The rheology modifiers are selected from the group consisting of non-polymeric crystalline, hydroxy-functional materials, polymeric rheology modifiers which impart shear thinning characteristics to the aqueous liquid matrix of a liquid detergent composition. The rheology and viscosity of the detergent can be modified and adjusted by methods known in the art, for example as shown in EP 2169040.

Other suitable adjunct materials include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, and structurants for liquid detergents and/or structure elasticizing agents.

### Formulation of detergent products

The detergent composition of the invention may be in any convenient form, e.g., a bar, a homogenous tablet, a tablet having two or more layers, a pouch having one or more compartments, a regular or compact powder, a granule, a paste, a gel, or a regular, compact or concentrated liquid.

A detergent pouch may be configured as single or multi compartments (see e.g., WO 2009/098660 or WO 2010/141301). It can be of any form, shape and material which is suitable for holding the detergent composition, e.g., without allowing release of the composition from the pouch prior to water contact. The pouch is made from water-soluble film which encloses the inner volume (detergent composition). Said inner volume can be divided into compartments of the pouch. The water-soluble film is described above under "Water-soluble film". The pouch can comprise a solid laundry cleaning (detergent) composition or selected components thereof, and/or a liquid cleaning composition or selected components thereof, separated by the water-soluble film. The pouch may include compartments having any combination of solids and liquids, both in one or more separate compartments, and in shared compartments containing both solid and liquid ingredients. The pouch may include regions or compartments formed by different water-soluble films, which can be with or without enzymes. Accordingly, detergent ingredients can be separated physically from each other in different compartments, or in different layers of a tablet if the detergent is in that physical form. Thereby negative storage interaction between components can be avoided. Different dissolution profiles of each of the compartments can also give rise to delayed dissolution of selected components in the wash solution.

A liquid or gel detergent, which is not unit dosed, may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent. A liquid or gel detergent may be non-aqueous.

### Compositions, methods and uses

The inventors of the present invention have provided water-soluble films with improved enzymatic storage stability and/or improved residual enzymatic activity.

Accordingly, in a first aspect, the present invention provides a water-soluble film comprising a protease and a protease inhibitor. Preferably, the protease inhibitor is capable of reducing the proteolytic activity of the protease. The inhibitor may have an inhibition constant to the protease, Kᵢ (mol/L), of from 1E-12 to 1E-03; more preferred from 1E-11 to 1E-04; even more preferred from 1E-10 to 1E-05; even more preferred from 1E-10 to 1E-06; and most preferred from 1E-09 to 1E-07.

Preferably the protease is a serine protease and the protease inhibitor is a serine protease inhibitor, more preferably the protease is a subtilisin and the protease inhibitor is a subtilisin protease inhibitor.

In an embodiment, the water-soluble film comprises one or more other enzymes selected from the group consisting of lipase, cutinase, amylase, carbohydrase, cellulase, pectinase, mannanase, arabinase, galactanase, xylanase, laccase, peroxidase and haloperoxidase.

In an embodiment, the protease inhibitor is a boronic acid derivative, preferably a phenyl boronic acid derivative, more preferably 4-formyl-phenyl-boronic acid (4-FPBA).

In another embodiment, the protease inhibitor is a peptide aldehyde protease inhibitor, preferably a hydrosulfite adduct of a peptide aldehyde protease inhibitor. Preferably, the peptide aldehyde protease inhibitor is Z-RAY-H, Ac-GAY-H, Z-GAY-H, Z-GAL-H, Z-GAF-H, Z-GAV-H, Z-RVY-H, Z-LVY-H, Ac-LGAY-H, Ac-FGAY-H, Ac-YGAY-H, Ac-FGVY-H, or Ac-WLVY-H; or a hydrosulfite adduct thereof; wherein Z is benzyloxycarbonyl and Ac is acetyl.

In an embodiment, the water-soluble film includes a salt of a monovalent cation and a monovalent organic anion of 1-6 carbons. Preferably, the monovalent organic anion is a small monocarboxylic acid of 1-6 carbons. The monovalent organic anion is preferably selected among formate, acetate, propionate and lactate. The cation may be Na⁺, K⁺ or NH₄⁺, and the salt may in particular be sodium formate.

In an embodiment, the water-soluble film comprises from 35% to 90% of PVOH which has a degree of hydrolysis of from 75% to 99%.

In an embodiment, the water-soluble film comprises from 10% to 50% of polyols.

In an embodiment, the water-soluble film has a thickness of from 10 µm to 500 µm.

In a second aspect, the invention provides a method for producing the water-soluble film described above, comprising: (a) adding a protease and a protease inhibitor to a liquid water-soluble film composition; and (b) forming a solid water-soluble film from the liquid composition. Preferably the protease is a serine protease, more preferably a subtilisin.

In an embodiment, one or more other enzymes selected from the group consisting of lipase, cutinase, amylase, carbohydrase, cellulase, pectinase, mannanase, arabinase, galactanase, xylanase, laccase, peroxidase and haloperoxidase are also added to the liquid composition in step (a).

In a third aspect, the invention provides a method for preparing a detergent unit dose product, comprising: (a) forming an enzymatic water-soluble film comprising a protease and a protease inhibitor, as described above; and (b) encapsulating a detergent composition in the enzymatic water-soluble film. Preferably the protease is a serine protease, more preferably a subtilisin.

In an embodiment, the detergent composition is a liquid laundry or dish wash detergent composition.

In another aspect, the invention provides a detergent pouch or unit dose product, comprising a compartment formed by a water-soluble film according to the invention, and a detergent composition containing a surfactant and/or a detergent builder. Preferably, the detergent composition is a liquid laundry or dish wash detergent composition.

In another aspect, the invention provides a detergent composition, comprising a surfactant and/or a detergent builder, and a water-soluble film comprising a protease and a protease inhibitor according to the invention and as described above.

In an embodiment, the surfactant and/or a detergent builder is encapsulated in the water-soluble film.

The invention also provides for use of the methods and compositions above for improving enzymatic storage stability and/or improving residual enzymatic activity in the water-soluble films described above.

The present invention is further described by the following examples that should not be construed as limiting the scope of the invention.

### EXAMPLE 1

We prepared two different enzyme containing water-soluble films, as outlined in Table 1 below. The films were produced under normal film production conditions. Later, the films were analyzed to estimate the enzymatic activity remaining after the production process, *i.e.,* the percentage of residual enzymatic activity in the final film product, as compared to the amount of enzyme added in the production process.

The protease used for preparing the films was Savinase (Novozymes A/S, Denmark), which was added to obtain a final concentration of 1.1% wt active enzyme protein in both of the water-soluble films.

The amylase used was Stainzyme (Novozymes A/S, Denmark), which was added to obtain a final concentration of 0.1 % wt active enzyme protein in both of the films.

The mannanase used was Mannaway (Novozymes A/S, Denmark), which was added to obtain a final concentration of 0.03% wt active enzyme protein in both of the films.

The protease inhibitor used was 4-formyl-phenyl-boronic acid (4-FPBA), which was added to obtain a final concentration of 0.5% wt in one of the water-soluble films.

Since the humidity of the samples was not controlled, some of the absolute values from the analysis are above 100%. However, all film samples were treated identically and analyzed at the same time, and therefore the relative improvement by using the protease inhibitor can be calculated.

**Table 1. The data are based on an average of three analyses of each film.**

| Enzyme | Residual activity in film made without 4-FPBA | Residual activity in film made with 4-FPBA | Relative improvement by using inhibitor |
|---|---|---|---|
| Protease | 87% | 93% | 7% |
| Amylase | 106% | 114% | 8% |
| Mannanase | 1% | 63% | ∼600% |

The data in Table 1 show quite clearly that the presence of a protease inhibitor during production of the film improves the survival of the enzymes in the process. This is of even higher importance if offcuts, fragments or leftovers of film are recycled in the process, to avoid discarding valuable material.

### EXAMPLE 2

A suitable detergent composition for encapsulation in a water-soluble film of the invention, thus forming a detergent pouch, may include the following ingredients:

| **Ingredient** | **Function** |
|---|---|
| linear alkylbenzene sulfonates | surfactant |
| C12-16 Pareth-9 | surfactant |
| alcoholethoxy sulfate | surfactant |
| fatty acid salts | surfactant |
| polyethyleneimine ethoxylate | polymer |
| glycerine | polymer |
| PEG-136 polyvinyl acetate | polymer |
| ethylene diamine disuccinic salt | chelant |
| diethylenetriamine pentaacetate, sodium | captures soil |
| propylene glycol | process aid |
| monoethanolamine citrate | process aid |
| sodium bisulfite | process aid |
| calcium formate | process aid |
| sodium formate | process aid |
| hydrogenated castor oil | process aid |
| mannanase | enzyme |
| xyloglucanase | enzyme |
| amylase | enzyme |
| perfume | fragrance |
| disodium distyrylbiphenyl disulfonate | brightener |
| dyes | colorant |
| benzisothiazolin | preservative |
| water | solvent |

### EXAMPLE 3

A suitable detergent composition for encapsulating in a water-soluble film of the invention (a detergent pouch) includes the following ingredients:

| |
|---|
| C12-14 Pareth-7 |
| MEA-dodecylbenzenesulfonate |
| MEA-laureth Sulfate |
| MEA-palm Kernelate |
| PEI ethoxylate |
| dodecylbenzene sulfonic acid |
| disodium distyrylbiphenyl disulfonate |
| PEG/PPG-10/2 propylheptyl ether |
| co-polymer of PEG / vinyl acetate |
| MEA citrate |
| potassium sulfite |
| magnesium chloride |
| hydrogenated castor oil |
| ethanolamine |
| polystyrene |
| enzymes |
| sodium formate |
| sorbitol |
| tripropylene glycol |
| 2-propenoic acid, polymer with ethenylbenzene |
| glycerin |
| propylene glycol |
| geraniol |
| eugenol |
| linalool |
| butylphenyl methylpropional |
| sulfuric acid |
| water |

### EXAMPLE 4

Other suitable detergent compositions for encapsulating in a water-soluble film of the invention (a detergent pouch) include the following common ingredients:

| | Detergent composition 1 | Detergent composition 2 | Detergent composition 3 | Detergent composition 4 |
|---|---|---|---|---|
| | wt% | wt% | wt% | wt% |
| LAS (C9-C15 alkylbenzene sulfonate) | 21 | 17 | 20 | 20 |
| sodium lauryl ether sulfate | 8 | 11 | 10 | 10 |
| non-ionic surfactant C13-7EO | 17 | 28 | 20 | 20 |
| fatty acid (cocoacid) | 15 | 4 | 5 | 10 |
| ethanolamine | 8 | 4 | 6 | 7 |
| citric acid | 2 | 2 | 2 | 2 |
| monopropylene glycol | 15 | 21 | 20 | 16 |
| glycerol | 6 | - | 7 | 5 |
| water | 8 | 13 | 10 | 10 |

The detergent compositions may also include enzymes, fragrances, color, brighteners, etc.

## Claims

1. A water-soluble film comprising a protease and a protease inhibitor.

2. The water-soluble film of claim 1, wherein the protease inhibitor is capable of reducing the proteolytic activity of the protease.

3. The water-soluble film of claims 1 or 2, wherein the protease is a serine protease and the protease inhibitor is a serine protease inhibitor.

4. The water-soluble film of any of claims 1-3, wherein the protease is a subtilisin and the protease inhibitor is a subtilisin inhibitor.

5. The water-soluble film of any of claims 1-4, wherein the water-soluble film comprises one or more other enzymes in addition to the protease.

6. The water-soluble film of any of claims 1-5, wherein the water-soluble film comprises a salt of formate.

7. The water-soluble film of any of claims 1-6, wherein the protease inhibitor is a peptide aldehyde protease inhibitor, preferably a hydrosulfite adduct of a peptide aldehyde protease inhibitor.

8. The water-soluble film of any of claims 1-7, wherein the peptide aldehyde protease inhibitor is Z-RAY-H, Ac-GAY-H, Z-GAY-H, Z-GAL-H, Z-GAF-H, Z-GAV-H, Z-RVY-H, Z-LVY-H, Ac-LGAY-H, Ac-FGAY-H, Ac-YGAY-H, Ac-FGVY-H, or Ac-WLVY-H; or a hydrosulfite adduct thereof; wherein Z is benzyloxycarbonyl and Ac is acetyl.

9. The water-soluble film of any of claims 1-8, wherein the protease inhibitor is boric acid, boronic acid, or a boronic acid derivative; preferably phenyl boronic acid or a phenyl boronic acid derivative, more preferably 4-formyl-phenyl-boronic acid (4-FPBA).

10. The water-soluble film of any of claims 1-9, comprising from 35% to 90% of PVOH which has a degree of hydrolysis of from 75% to 99%.

11. The water-soluble film of any of claims 1-10, comprising from 10% to 50% of polyols.

12. The water-soluble film of any of claims 1-11, which has a thickness of from 10 µm to 500 µm.

13. A method for producing a water-soluble film according to any of claims 1-12, comprising:
(a) adding a protease and a protease inhibitor to a liquid water-soluble film composition; and
(b) forming a solid water-soluble film from the liquid composition.

14. A method for preparing a detergent unit dose product, comprising:
(a) forming an enzymatic water-soluble film according to any of claims 1-12; and
(b) encapsulating a detergent composition in the enzymatic water-soluble film.

15. The method of claim 14, wherein the detergent composition is a liquid laundry or dish wash detergent composition.

16. A detergent pouch or unit dose product, comprising a compartment formed by a water-soluble film according to any of claims 1-12, and a detergent composition containing a surfactant and/or a detergent builder.

## Patentansprüche

1. Wasserlöslicher Film, der eine Protease und einen Proteaseinhibitor umfasst.

2. Wasserlöslicher Film nach Anspruch 1, wobei der Proteaseinhibitor zum Verringern der proteolytischen Aktivität der Protease fähig ist.

3. Wasserlöslicher Film nach Ansprüchen 1 oder 2, wobei die Protease eine Serinprotease ist, und der Proteaseinhibitor ein Serinproteaseinhibitor ist.

4. Wasserlöslicher Film nach einem beliebigen der Ansprüche 1-3, wobei die Protease ein Subtilisin ist, und der Proteaseinhibitor ein Subtilisininhibitor ist.

5. Wasserlöslicher Film nach einem beliebigen der Ansprüche 1-4, wobei der wasserlösliche Film ein oder mehrere andere Enzyme zusätzlich zur Protease umfasst.

6. Wasserlöslicher Film nach einem beliebigen der Ansprüche 1-5, wobei der wasserlösliche Film ein Salz von Formiat umfasst.

7. Wasserlöslicher Film nach einem beliebigen der Ansprüche 1-6, wobei der Proteaseinhibitor ein Peptidaldehyd-Proteaseinhibitor ist, bevorzugt ein Hydrosulfitaddukt eines Peptidaldehyd-Proteaseinhibitors.

8. Wasserlöslicher Film nach einem beliebigen der Ansprüche 1-7, wobei der Peptidaldehyd-Proteaseinhibitor Z-RAY-H, Ac-GAY-H, Z-GAY-H, Z-GAL-H, Z-GAF-H, Z-GAV-H, Z-RVY-H, Z-LVY-H, Ac-LGAY-H, Ac-FGAY-H, Ac-YGAY-H, Ac-FGVY-H oder Ac-WLVY-H ist; oder ein Hydrosulfitaddukt davon; wobei Z Benzyloxycarbonyl ist und Ac Acetyl ist.

9. Wasserlöslicher Film nach einem beliebigen der Ansprüche 1-8, wobei der Proteaseinhibitor Borsäure, Boronsäure oder ein Boronsäurederivat; bevorzugt Phenylboronsäure oder ein Phenylboronsäurederivat, stärker bevorzugt 4-Formylphenylboronsäure (4-FPBA) ist.

10. Wasserlöslicher Film nach einem beliebigen der Ansprüche 1-9, der von 35% bis 90% PVOH umfasst, das einen Grad der Hydrolyse von 75% bis 99% aufweist.

11. Wasserlöslicher Film nach einem beliebigen der Ansprüche 1-10, der von 10% bis 50% Polyole umfasst.

12. Wasserlöslicher Film nach einem beliebigen der Ansprüche 1-11, der eine Dicke von 10 µm bis 500 µm aufweist.

13. Verfahren zum Herstellen eines wasserlöslichen Films gemäß einem beliebigen der Ansprüche 1-12, umfassend:
a) Zugeben einer Protease und eines Proteaseinhibitors zu einer flüssigen wasserlöslichen Filmzusammensetzung; und
b) Bilden eines festen wasserlöslichen Films aus der flüssigen Zusammensetzung.

14. Verfahren zum Herstellen eines Detergens-Einheitsdosisprodukts, umfassend:
a) Bilden eines enzymatischen wasserlöslichen Films gemäß einem beliebigen der Ansprüche 1-12; und
b) Verkapseln einer Detergenszusammensetzung im enzymatischen wasserlöslichen Film.

15. Verfahren nach Anspruch 14, wobei die Detergenszusammensetzung eine Flüssigwaschmittel- oder Geschirrspül-Detergenszusammensetzung ist.

16. Detergensbeutel oder Einheitsdosisprodukt, das eine Kammer, die durch einen wasserlöslichen Film gemäß einem beliebigen der Ansprüche 1-12 gebildet ist, und eine Detergenszusammensetzung, die ein oberflächenaktives Mittel und/oder einen Detergens-Gerüststoff enthält, umfasst.

## Revendications

1. Film soluble dans l'eau comprenant une protéase et un inhibiteur de protéase.

2. Film soluble dans l'eau selon la revendication 1, dans lequel l'inhibiteur de protéase est capable de réduire l'activité protéolytique de la protéase.

3. Film soluble dans l'eau selon la revendication 1 ou 2, dans lequel la protéase est une sérine protéase et l'inhibiteur de protéase est un inhibiteur de sérine protéase.

4. Film soluble dans l'eau selon l'une quelconque des revendications 1 à 3, dans lequel la protéase est une subtilisine et l'inhibiteur de protéase est un inhibiteur de subtilisine.

5. Film soluble dans l'eau selon l'une quelconque des revendications 1 à 4, dans lequel le film soluble dans l'eau comprend une ou plusieurs autres enzymes en plus de la protéase.

6. Film soluble dans l'eau selon l'une quelconque des revendications 1 à 5, dans lequel le film soluble dans l'eau comprend un sel formiate.

7. Film soluble dans l'eau selon l'une quelconque des revendications 1 à 6, dans lequel l'inhibiteur de protéase est un inhibiteur d'aldéhyde protéase peptidique, de préférence un adduit hydrosulfite d'un inhibiteur d'aldéhyde protéase peptidique.

8. Film soluble dans l'eau selon l'une quelconque des revendications 1 à 7, dans lequel l'inhibiteur d'aldéhyde protéase peptidique est Z-RAY-H, Ac-GAY-H, Z-GAY-H, Z-GAL-H, Z-GAF-H, Z-GAV-H, Z-RVY-H, Z-LVY-H, Ac-LGAY-H, Ac-FGAY-H, Ac-YGAY-H, Ac-FGVY-H, ou Ac-WLVY-H ; ou un adduit hydrosulfite de celui-ci ; dans lequel Z est benzyloxycarbonyle et Ac est acétyle.

9. Film soluble dans l'eau selon l'une quelconque des revendications 1 à 8, dans lequel l'inhibiteur de protéase est l'acide borique, l'acide boronique, ou un dérivé d'acide boronique ; de préférence l'acide phénylboronique ou un dérivé d'acide phénylboronique, plus préférablement l'acide 4-formyl-phényl-boronique (4-FPBA).

10. Film soluble dans l'eau selon l'une quelconque des revendications 1 à 9, comprenant de 35 % à 90 % de PVOH qui a un degré d'hydrolyse de 75 % à 99 %.

11. Film soluble dans l'eau selon l'une quelconque des revendications 1 à 10, comprenant de 10 % à 50 % de polyols.

12. Film soluble dans l'eau selon l'une quelconque des revendications 1 à 11, qui a une épaisseur de 10 µm à 500 µm.

13. Méthode de production d'un film soluble dans l'eau selon l'une quelconque des revendications 1 à 12, comprenant :
(a) l'addition d'une protéase et d'un inhibiteur de protéase à une composition liquide de film soluble dans l'eau ; et
(b) la formation d'un film soluble dans l'eau solide à partir de la composition liquide.

14. Méthode de préparation d'un produit détergent en dose unitaire, comprenant :
(a) la formation d'un film soluble dans l'eau enzymatique selon l'une quelconque des revendications 1 à 12 ; et
(b) l'encapsulation d'une composition détergente dans le film soluble dans l'eau enzymatique.

15. Méthode selon la revendication 14, dans laquelle la composition détergente est une composition détergente liquide pour le lavage du linge ou de la vaisselle.

16. Produit en dose unitaire ou sachet de détergent, comprenant un compartiment formé par un film soluble dans l'eau selon l'une quelconque des revendications 1 à 12, et une composition détergente contenant un tensioactif et/ou un adjuvant pour détergent.
